# EUROPÄISCHE PATENTSCHRIFT

(11) **EP 2 786 696 B1**
(45) Veröffentlichungstag und Bekanntmachung des Hinweises auf die Patenterteilung: **11.07.2018**
(21) Anmeldenummer: 14001225.3
(22) Anmeldetag: 02.04.2014
(51) Int. Cl.: A61B 1/24, A61B 5/107, A61C 9/00, A61B 1/00, A61B 1/04, H04N 5/225

(54) **Dentalkamerasystem**
Dental camera system
Système de caméra dentaire

(30) Priorität: 04.04.2013 DE 102013005616
(43) Veröffentlichungstag der Anmeldung: 08.10.2014
(73) Patentinhaber: Dürr Dental SE, 74321 Bietigheim-Bissingen (DE)
(72) Erfinder: Lais, Peter, 74391 Erligheim (DE); Maier, Raimund, 71732 Tamm (DE); Hack, Alexander, 88400 Biberach (DE); Schramm, Axel, 74360 Ilsfeld (DE)
(74) Vertreter: Ostertag & Partner Patentanwälte mbB

(56) Entgegenhaltungen:
- WO-A1-2009/144729
- JP-A- 2005 069 936
- US-A1- 2012 281 072
- ARASH TAHERI ET AL: "Focusing and depth of field in photography: application in dermatology practice", SKIN RESEARCH AND TECHNOLOGY, Bd. 19, Nr. 4, 25. März 2013 (2013-03-25), Seiten n/a-n/a, XP055122099, ISSN: 0909-752X, DOI: 10.1111/srt.12058
- REN NG ET AL: "Light Field Photography with a Hand-held Plenoptic Camera", STANDFORD TECH REPORT CTSR,, 1. Februar 2005 (2005-02-01), Seiten 1-11, XP008138449,

## Beschreibung

### 1. Gebiet der Erfindung

Die Erfindung betrifft ein Dentalkamerasystem zum Erzeugen einer Darstellung eines Zahns oder eines anderen Objektes.

### 2. Beschreibung des Standes der Technik

Dentalkamerasysteme werden in der Zahnmedizin für Aufnahmen des Mundes, insbesondere des Mundinnenraumes und der darin angeordneten Zähne, verwendet. Die dabei entstehenden Darstellungen kann der behandelnde Arzt zum Zwecke der Diagnose oder zur Besprechung von Behandlungsmöglichkeiten mit dem Patienten auf einem Anzeigegerät anzeigen lassen. Zudem kann der Arzt die Darstellungen einer Langzeitarchivierung zuführen, um den Behandlungsverlauf zu dokumentieren. Eine in solchen Systemen verwendete Dentalkamera ist beispielsweise aus der DE 10 2009 017 819 A1 bekannt.

Des Weiteren sind Dentalkamerasysteme bekannt, die über eine konventionelle abbildende Funktion hinaus weitere diagnostische Möglichkeiten eröffnen. So sind beispielsweise Dentalkamerasysteme bekannt, bei welchen mit Hilfe eines meist im UV-Bereich liegenden Anregungslichts Kariesbakterien oder mit diesen verbundene Fluoreszenzmarker zu Fluoreszenz angeregt werden. Durch Messung des daraufhin emittierten Fluoreszenzlichts lässt sich vorhandener Kariesbefall lokalisiert nachweisen, was beispielsweise eine Zahnsubstanz schonendere Behandlung erlaubt.

Wie aus der oben erwähnten DE 10 2009 017 819 A1 hervorgeht, haben insbesondere Dentalkameras mit hoher Bildqualität meist eine aufwendige Optik mit mehreren Linsen. Darüber hinaus müssen derartige Dentalkameras einzelne bewegliche Komponenten, wie beispielsweise eine verschiebbare Linse oder einen verschiebbaren Bildwandler, aufweisen, um bei der Abbildung auf den Bildwandler eine Verstellbarkeit der Objektweite zu ermöglichen. Eine andere Möglichkeit zur Verstellung der Objektweite besteht in der Verwendung von Flüssiglinsen, bei welchen die Krümmung einer Grenzschicht zwischen zwei Flüssigkeiten verändert wird, wodurch sich die Brechkraft der Flüssiglinse verändert.

Nachteilig an diesen bekannten Dentalkameras ist, dass die Optik aufwendig und groß ist, wodurch auch der in den Mundinnenraum einzuführende Kamerakopf eine gewisse Mindestgröße nicht unterschreiten kann. Zudem müssen für das präzise Verschieben der beweglichen Komponenten mechanisch aufwendige Antriebe verwendet werden, die eine Steuerelektronik erfordern. Ähnliches gilt für Flüssiglinsen, für welche ebenfalls eine Steuerelektronik notwendig ist.

Darüber hinaus ist aus der US 2012/0281072 A1 eine fokussierende plenoptische Kamera mit mehreren Abbildungskanälen bekannt, bei der ein Filter vorgesehen ist, das in unterschiedlichen Abbildungskanälen unterschiedliche Filterwirkungen bewirkt.

Aus der JP 2005 069936 A ist bekannt, eine Lichtfeldkamera im Medizinbereich als Endoskop zu verwenden.

Ferner ist aus ARASH TAHERI ET AL: "Focusing and depth of field in photography: application in dermatology practice", SKIN RESEARCH AN TECHNOLOGY, Bd. 19, Nr. 4, 2013-03-25, bekannt in der Dermatologie eine Lichtfeldkamera zur Aufnahme von Mundverletzungen zu verwenden.

### ZUSAMMENFASSUNG DER ERFINDUNG

Es ist daher Aufgabe der Erfindung, ein Dentalkamerasystem anzugeben, bei welchem die Optik der Dentalkamera ohne bewegliche oder ansteuerbare optische Elemente auskommt und dennoch ein großer Objektweitenbereich scharf dargestellt werden kann.

Erfindungsgemäß wird dies durch ein Dentalkamerasystem mit einer Dentalkamera erreicht, die ein Gehäuse, eine darin angeordnete Optik und einen Bildwandler aufweist. Das Dentalkamerasystem umfasst ferner eine Auswerteeinheit, die mit dem Bildwandler zusammenarbeitet, um das Objekt zu erfassen. Die Optik ist erfindungsgemäß eine plenoptische Optik, die dazu eingerichtet ist, beim Erfassen des Objektes ein 4D-Lichtfeld zu bestimmen. Die Auswerteeinheit ist dazu eingerichtet, aus dem 4D-Lichtfeld mindestens eine Darstellung des erfassten Objekts zu berechnen.

Die Erfinder haben erkannt, dass sich für eine Dentalkamera, insbesondere für eine Intraoralkamera, eine kompakt bauende Optik hoher Variabilität durch eine plenoptische Optik realisieren lässt.

Konventionelle Kameras bilden den 3D-Raum des Objekts auf ein 2D-Bild ab, welches mit dem Bildwandler erfasst wird. Ein solches 2D-Bild enthält keine Informationen darüber, aus welcher Richtung ein vom Bildwandler erfasster Lichtstrahl kam. Im Gegensatz dazu erfassen plenoptische Kameras mit plenoptischen Optiken, die als solche beispielsweise aus der Druckschrift US 8,265,478 B1 bekannt sind, zusätzlich zur Position und Intensität eines auf den Bildwandler fallenden Lichtstrahls auch dessen Richtung und ermöglichen so, das 4D-Lichtfeld eines Objektes zu erfassen. Da das 4D-Lichtfeld, auch plenoptische Funktion oder Brillianz genannt, umfassende Informationen über die optischen Verhältnisse des erfassten Objektes repräsentiert, ist es mit Hilfe eines in der Auswerteeinheit hinterlegten Auswertealgorithmus möglich, aus diesem 4D-Lichtfeld eine einem üblichen 2D-Bild entsprechende Darstellung zu berechnen.

Wie im Folgenden im Hinblick auf verschiedene Weiterbildungen der Erfindungen deutlich werden wird, können dabei nachträglich nach der Aufnahme über den Bildwandler allein durch Ändern verschiedener Parameter des Auswertealgorithmus oder des Auswertealgorithmus selbst verschiedene Abbildungseigenschaften, wie beispielsweise die Objektweite, geändert werden, ohne dass hierzu bewegliche oder ansteuerbare optische Elemente in der Dentalkamera notwendig sind.

Kennzeichnend für eine plenoptische Kamera ist, dass die Optik ein Array von optischen Elementen aufweist, das den Strahlengang der Optik in mehrere nebeneinander angeordnete Abbildungskanäle unterteilt, deren Sichtfelder sich teilweise überlappen. Jeder optische Kanal enthält also ein oder mehrere optische Elemente. Eine teilweise Überlappung der Sichtfelder ist notwendig, damit für jeden Punkt des Objekts Informationen in mehreren Abbildungskanälen vorhanden sind, so dass das 4D-Lichtfeld erfasst werden kann. Ein Array, d.h. eine Rasteranordnung, von optischen Elementen erlaubt es, auf einfache Weise den Strahlengang der Optik insgesamt in mehrere derartige Abbildungskanäle zu unterteilen. Hierfür kann beispielsweise ein Lochblendenarray oder ein Array von Fresnelzonen- oder Stufenlinsen verwendet werden.

Erfindungsgemäß ist dabei vorgesehen, dass das Array von optischen Elementen ein Linsenarray ist, das mehrere Linsen umfasst, wobei jede Linse einem der Abbildungskanäle zugeordnet ist.

Ein Linsenarray, insbesondere ein aus mehreren sehr kleinen Linsen (Durchmesser ca. 10 µm - 30 µm) gefertigtes Mikrolinsenarray, ist gegenüber einem Lochblendenarray vorteilhaft, da es eine bessere Lichtausbeute bietet. Da jede der Linsen in dem Linsenarray das Objekt unter einem leicht unterschiedlichen Blickwinkel erfasst, entsteht eine Abbildung des Objektes auf dem Bildwandler innerhalb eines einem Abbildungskanal zugeordneten Bildwandlerbereichs an leicht lateral zueinander verschobenen Orten. Der Auswertealgorithmus der Auswerteeinheit wertet diese Verschiebungen bei der Berechnung der Darstellung des Objekts aus.

Erfindungsgemäß sind die Linsen des Linsenarrays die einzigen Linsen der Optik.

Die Optik der Dentalkamera erhält dadurch einen einfacheren Aufbau. Die vorzugsweise sammelnde Brechkraft der Linsen des Linsenarrays kann dabei so gewählt werden, dass das Linsenarray in direkter Nachbarschaft des Bildwandlers oder in direktem Kontakt mit diesem angeordnet werden kann. Auf diese Weise lässt sich eine Dentalkamera mit einem sehr kompakten Kamerakopf herstellen. Die Tatsache, dass entlang des Strahlengangs keine weiteren Linsen außer den Linsen des Linsenarrays vorgesehen sind, macht zudem die ansonsten notwendige präzise Ausrichtung verschiedener Linsen zueinander überflüssig. Andere optische Elemente ohne sammelnde oder streuende Wirkung wie beispielsweise ein Eintrittsfenster oder ein Filter können jedoch noch im Strahlengang der Optik enthalten sein.

Erfindungsgemäß weisen dabei einzelne Linsen oder Gruppen von Linsen des Linsenarrays von den anderen Linsen abweichende Brechkraft auf.

Ein solches Linsenarray mit Linsen unterschiedlicher Brechkraft ist im Hinblick auf das Berechnen der Darstellungen des Objekts mit unterschiedlichen Objektweiten vorteilhaft sein. Denn dadurch wird der Bereich der Objektweiten vergrößert, in welchem Darstellungen berechnet werden können, die ausreichend scharf sind. Vorzugsweise wird ein relativ großer Bereich von ca. 1 mm bis ca. 300 mm Objektweite abgedeckt, so dass die Dentalkamera ohne bewegliche Teile sowohl für Makroaufnahmen einzelner Zähne als auch für Gesichtsaufnahmen des Patienten verwendet werden kann.

In einer Ausgestaltung der Erfindung umfassen die Abbildungskanäle eine erste Gruppe von Abbildungskanälen und eine zweite Gruppe von Abbildungskanälen. Im Strahlengang der Abbildungskanäle der ersten Gruppe ist jeweils ein Filter mit einer ersten Filterwirkung und im Strahlengang der Abbildungskanäle der zweiten Gruppe kein Filter oder ein Filter mit einer zweiten Filterwirkung angeordnet, die sich von der ersten Filterwirkung unterscheidet.

Dies ermöglicht beispielsweise die Verwendung des Dentalkamerasystems zu Fluoreszenzmesszwecken. So kann die erste Filterwirkung der ersten Gruppe von Abbildungskanälen, welche eine echte Untermenge aller Abbildungskanäle darstellt, beispielsweise so gewählt werden, dass nur Fluoreszenzlicht passieren kann, welches von dem erfassten Objekt durch Beleuchten mit Anregungslicht emittiert wird. Die Filterwirkung wird dabei so gewählt, dass im betreffenden Abbildungskanal vom Bildwandler nur Wellenlängen größer als ca. 495 nm erfasst werden. Dabei kann beispielsweise nur jeder zehnte Abbildungskanal über ein Filter mit einer derartige Filterwirkung verfügen. Eine solche Optik ermöglicht einerseits die Aufnahme konventioneller farbechter Aufnahmen sowie Fluoreszenzlichtmessungen, ohne dass dabei ein bewegliches Filter in den Strahlengang eingefügt oder aus diesem heraus genommen werden muss. Bei ausreichender Intensität des Fluoreszenzlichts und entsprechender Empfindlichkeit des Bildwandlers kann ein konventionelles Bild und ein Fluoreszenzbild gleichzeitig aufgenommen werden. Die jeweiligen Filter im Strahlengang können über eine Filtermaske vor oder hinter dem Array von optischen Elementen im Strahlengang angeordnet werden. Vorzugsweise wird dabei mindestens ein Filter mit der ersten Filterwirkung durch eine Linse des Linsenarrays gebildet.

Dadurch muss im Strahlengang kein weiteres Bauteil als Filter vorgesehen werden. Zur Herstellung eines solchen Linsenarrays können einzelne Linsen beispielsweise durch Beschichten, Einfärben oder Dotieren mit einer Filterwirkung versehen werden, die sich von den anderen Linsen unterscheidet.

Alternativ oder zusätzlich hierzu kann der Bildwandler selbst eine Filtermaske aufweisen, die in ausgewählten Bereichen des Bildwandlers eine wellenlängenabhängige Empfindlichkeit erzeugt.

In einer Ausgestaltung der Erfindung ist die plenoptische Optik als fokussierte plenoptische Optik ausgebildet.

Dies hat den Vorteil, dass eine für Dentalaufnahmen ausreichende Auflösung erreicht werden kann. Insbesondere werden die Bildweite von dem Linsenarray zum Bildwandler und die Brechkraft der Linsen derart gewählt, dass eine in einem für Dentalkamerasysteme üblichen Arbeitsabstand von ca. 5 mm bis ca. 15 mm, vorzugsweise ca. 12 mm, liegende Ebene des Objekts scharf auf dem Bildwandler abgebildet wird.

In einer Ausgestaltung der Erfindung ist die Auswerteeinheit dazu eingerichtet, aus dem 4D-Lichtfeld Darstellungen des Objekts mit unterschiedlichen Objektweiten zu berechnen.

Hierzu verwendet die Auswerteeinheit einen als solchen bekannten 4D-Lichtfeld-Auswertealgorithmus, in welchem die Objektweite als Parameter berücksichtigt wird. Über ein Eingabegerät, beispielsweise über eine Wippentaste an der Dentalkamera, kann der Benutzer dabei die gewünschte Objektweite vorgeben, was einer Veränderung der Brennweite bei einem konventionellen Dentalkamerasystem entspricht. Bei einer Echtzeit-Auswertung des 4D-Lichtfeldes kann direkt der die Objektweite definierende Parameter verändert werden, so dass die Darstellungen jeweils mit der momentan gewünschten Objektweite berechnet werden. Der Auswertealgorithmus kann aber auch zunächst mehrere Darstellungen mit unterschiedlichen Objektweiten berechnen und diese im Speicher hinterlegen. Der Benutzer kann dann nachträglich die Darstellung mit der gewünschten Objektweite auswählen. Bei entsprechender Ausgestaltung der plenoptischen Optik kann die gewünschte Objektweite dabei in einem für Dentalkameras üblichen Bereich zwischen ca. 1 mm und ca. 300 mm, insbesondere zwischen ca. 1 mm und ca. 55 mm, insbesondere zwischen ca. 5 und ca. 45 mm frei gewählt werden.

In einer Ausgestaltung der Erfindung ist die Auswerteeinheit dazu eingerichtet, aus den Darstellungen mit unterschiedlichen Objektweiten anhand mindestens eines vorgebbaren Bewertungskriteriums automatisch eine Darstellung auszuwählen.

Mit Hilfe eines Bewertungskriteriums, das den Schärfeeindruck einer Darstellung bewertet, kann aus den Darstellungen mit unterschiedlichen Objektweiten automatisch diejenige ausgewählt.werden, welche für den Benutzer besonders scharf erscheint. Die ausgewählte Darstellung wird dann auf einem Anzeigegerät dargestellt. Auf diese Weise lässt sich noch nach der Erfassung des Objekts rein rechnerisch eine Art Autofokus realisieren.

Insbesondere kann hierbei vorgesehen sein, dass das mindestens eine Bewertungskriterium ein Ergebnis einer Kontrastmessung in zumindest einem Teilbereich der Darstellungen umfasst.

Der Schärfeeindruck einer Darstellung kann durch eine Kontrastmessung, insbesondere eine Kantenkontrastmessung, bei der Bildfrequenzanteile oder Kantendicken bestimmt werden, bewertet werden. Über ein Eingabegerät kann dabei der Teilbereich der Darstellungen (Region-of-Interest) festgelegt werden, in welchem das Optimum des Schärfeeindrucks bestimmt werden soll.

In einer Ausgestaltung der Erfindung ist die Auswerteeinheit dazu eingerichtet, aus dem 4D-Lichtbild Darstellungen des Objekts mit unterschiedlichem Beobachterstandpunkt zu berechnen.

Aufgrund der verschiedenen nebeneinander angeordneten Abbildungskanäle kann der Beobachterstandpunkt bei entsprechender Parametrisierung des Auswertealgorithmus in gewissen Grenzen frei gewählt werden. So lassen sich Darstellungen des Objekts mit unterschiedlichem Beobachterstandpunkt berechnen und darstellen.

Vorzugsweise ist dabei vorgesehen, dass die Auswerteeinheit ein 3D-Anzeigegerät, insbesondere einen autostereoskopischen Bildschirm, aufweist und dazu eingerichtet ist, unter Verwendung mindestens zwei der Darstellungen mit unterschiedlichem Beobachterstandpunkt das erfasste Objekt dreidimensional darzustellen.

Dies ermöglicht es, ein Objekt, beispielsweise einen Zahn, räumlich darzustellen, indem für das linke Auge eine Darstellung aus Sicht eines "linken" Beobachterstandpunkts und für das rechte Auge eine Darstellung aus Sicht eines "rechten" Beobachterstandpunkts gewählt wird. Auch hier kann über ein Eingabegerät der Abstand zwischen den beiden Beobachterstandpunkten gewählt werden, was eine Art dreidimensionaler Zoom auf das Objekt ermöglicht bzw. das Einstellen der Eigenschaften der 3D-Anzeige erlaubt.

Die Auswerteeinheit kann ferner dazu eingerichtet sein, mit Hilfe des 4D-Lichtfeldes das Objekt dreidimensional zu vermessen.

Aufgrund der vollumfänglichen Informationen, die in dem erfassten 4D-Lichtfeld enthalten sind, ist es mit Hilfe eines Tiefenauswertealgorithmus in der Auswerteeinheit möglich, das erfasste Objekt dreidimensional zu vermessen. Dies bedeutet, dass man beispielsweise von einer Bissfläche eines Zahns ein dreidimensionales Modell erstellen kann, was gegebenenfalls einen klassischen mechanischen Abdruck der Bissfläche ersetzt. Diese Ausgestaltung der Erfindung stellt insofern eine wesentliche Neuerung dar, da bisherige System zur dreidimensionalen Vermessung von Zähnen stets in separaten Diagnosegeräten mit Hilfe von Laserscannern realisiert wurden. Vorzugsweise kann bei der vorliegenden Erfindung zur Unterstützung der dreidimensionalen Vermessung das zu erfassende Objekt mit Hilfe einer Lichtquelle mit einem vorgegebenen Lichtmuster beleuchtet werden. Hierzu kann beispielsweise eine LED mit einer Maske verwendet werden, die ein Streifen- oder Punktmuster auf dem beleuchteten Objekt erzeugt.

### KURZE BESCHREIBUNG DER ZEICHNUNGEN

Nachstehend wird die Erfindung anhand von Ausführungsbeispielen unter Bezugnahme auf die Zeichnungen erläutert. Darin zeigen:
- Figur 1: eine schematische Darstellung des erfindungsgemäßen Dentalkamerasystems mit einer Dentalkamera und einer Auswerteeinheit;
- Figur 2: eine perspektivische Ansicht der Dentalkamera von der Unterseite;
- Figur 3: einen Längsschnitt durch einen Kopfabschnitt der Dentalkamera;
- Figur 4: eine perspektivische Ansicht eines Mikrolinsenarrays, das vor einem Bildwandler der Dentalkamera angeordnet ist;
- Figur 5: eine schematische Darstellung der bei der erfindungsgemäßen Dentalkamera verwendeten plenoptischen Optik.

### BESCHREIBUNG BEVORZUGTER AUSFÜHRUNGSBEISPIELE

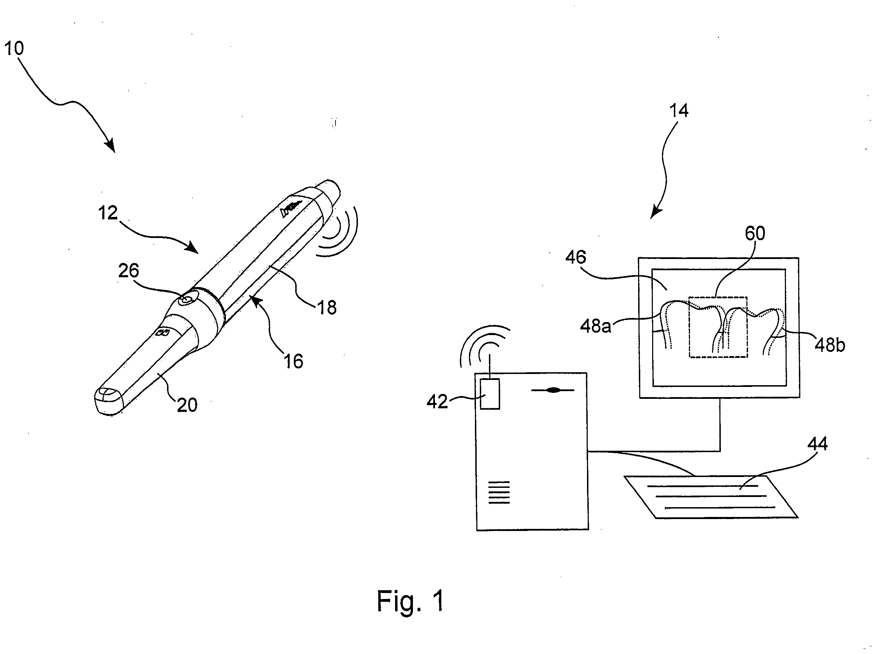

Figur 1 zeigt ein insgesamt mit 10 bezeichnetes Dentalkamerasystem mit einer Dentalkamera 12 und einer Auswerteeinheit 14.

Die Dentalkamera 12 weist ein Gehäuse 16 auf, das sich im Wesentlichen entlang einer Längsachse erstreckt und einen Griffabschnitt 18 umfasst, an welchem der untersuchende Arzt die Dentalkamera 12 führt. Am aus Sicht des Arztes distalen Ende des Gehäuses 16 ist ein Kopfabschnitt 20 angeordnet, der schlanker als der Griffabschnitt 18 ausgebildet ist und in die Mundöffnung eines Patienten eingeführt wird.

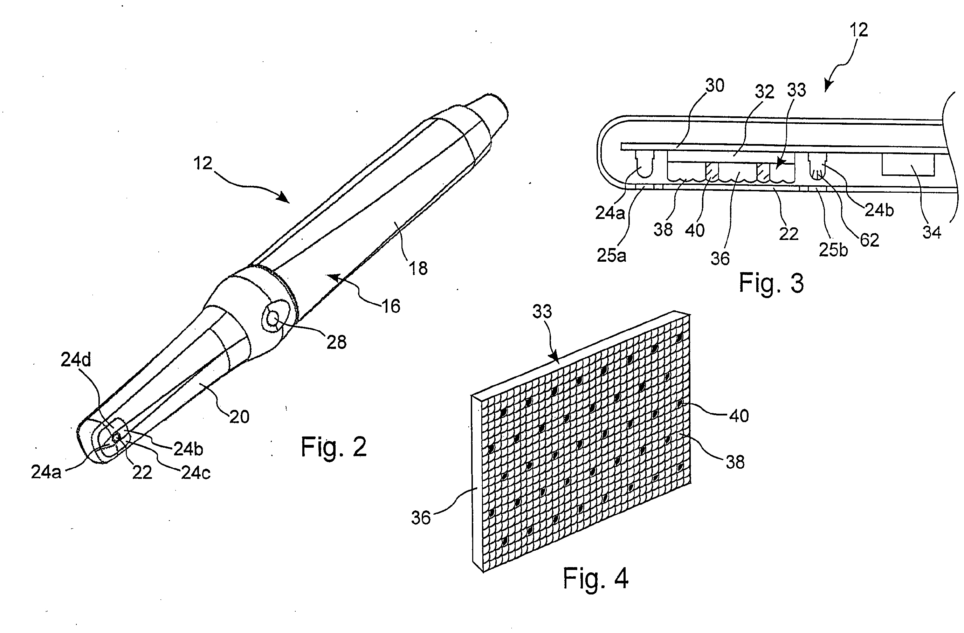

Wie aus Figur 2 ersichtlich, ist am Ende des Kopfabschnitts 20 ein Eintrittsfenster 22 angeordnet, welches durch Positionieren und Drehen der Dentalkamera 12 in Richtung eines zu erfassenden Objektes, beispielsweise eines Zahnes, ausgerichtet werden kann. Um das Eintrittsfenster 22 herum sind verschiedene Beleuchtungseinrichtungen 24a bis 24d angeordnet, welche weiter unten im Detail beschrieben werden.

Zur Aktivierung verschiedener Funktionen weist der Griffabschnitt 18 ferner bezüglich der Längsachse der Dentalkamera 12 diametral gegenüberliegend einen Standbildschalter 26 und einen Moduswahlschalter 28 als Betätigungsschalter auf.

Wie in Figur 3 gezeigt, ist im Inneren des Gehäuses 16 eine Platine 30 angeordnet, welche entlang der Längsachse verläuft. Auf dieser Platine 30 ist im Bereich hinter dem Eingangsfenster 22 ein Bildwandler 32 angeordnet, der über die Platine 30 elektrisch mit einem Steuer- und Kommunikationsmodul 34 verbunden ist, über welches die Dentalkamera 12 mit der Auswerteeinheit 14 beispielsweise per WLAN in Verbindung steht.

Um den Bildwandler 32 herum sind ebenfalls direkt auf der Platine 30 die Beleuchtungseinrichtungen 24a bis 24d angeordnet, von welchen im Längsschnitt die beiden Beleuchtungseinrichtungen 24a und 24b in Form von LEDs jeweils hinter Austrittsfenstern 25a und 25b zu erkennen sind. Auch die Beleuchtungseinrichtungen 24a bis 24d sind elektrisch mit dem Steuer- und Kommunikationsmodul 34 verbunden, welches die Beleuchtungseinrichtungen 24a bis 24d getrennt voneinander an- und ausschalten kann.

Die elektrischen Systeme der Dentalkamera 12 werden über einen nicht gezeigten Energiespeicher im Griffabschnitt 18 mit Energie versorgt.

Wie man der Figur 3 weiter entnehmen kann, trägt der Bildwandler 32 als plenoptische Optik 33 ein Linsenarray 36, das mehrere rasterförmig nebeneinander angeordnete Linsen 38 aufweist. Die plenoptische Optik 33 umfasst ferner nicht gezeigte Blenden mit fester Blendenöffnung, die den einzelnen Linsen 38 des Linsenarrays 36 zugeordnet sind. Dazu kann eine Blendenmaske aus undurchsichtigem Material in einer Art kompaktem Schichtsystem in dem im Wesentlichen aus durchsichtigem Material gefertigten Linsenarray 36 gekapselt werden.

Wie aus der perspektivischen Darstellung in Figur 4 ersichtlich ist, weisen einzelne der Linsen 38, hier als Filterlinsen 40 bezeichnet, eine von den anderen Linsen 38 abweichende Filterwirkung auf, was durch eine entsprechende Einfärbung der Filterlinsen 40 schematisch dargestellt ist. Diese Filterwirkung kann durch Beschichten, Einfärben oder Dotieren der einzelnen Filterlinsen 40 erreicht werden oder kann in Anlehnung an die Blendenmaske über eine Filtermaske realisiert sein, die mit dem Linsenarray 36 gekapselt ist..

Die hier als handelsüblicher PC ausgeführte Auswerteeinheit 14 steht, wie die Figur 1 zeigt, über ein Kommunikationsmodul 42 in Kommunikationsverbindung mit dem Steuer- und Kommunikationsmodul 34 der Dentalkamera 12, sodass die von dem Bildwandler 32 erfassten Bilddaten gegebenenfalls unter Zuhilfenahme geeigneter Komprimierverfahren an die Auswerteeinheit 14 übermittelt werden.

Die hier gezeigte Auswerteeinheit 14 umfasst ferner eine Tastatur 44 als Eingabegerät, wobei man jedoch auch speziell für die Verwendung in einem Dentalkamerasystem 10 optimierte Eingabegeräte, die dem Benutzer nur wenige unbedingt notwendige Eingaberegler zur Verfügung stellen, vorsehen kann. Zusätzliche Eingabesignale erhält die Auswerteeinheit 14 von dem Standbildschalter 26 und dem Moduswahlschalter 28 der Dentalkamera 12, deren Schaltzustand wie die Bilddaten über die Kommunikationsverbindung übermittelt wird.

Schließlich weist die Auswerteeinheit 14 einen autostereoskopischen Bildschirm 46 als 3D-Anzeigegerät auf. Autostereoskopische Bildschirme 46 sind im Stand der Technik bekannt und können beispielsweise unter Verwendung einer Zylinderlinsenmaske für die unterschiedlichen Blickrichtungen des linken und des rechten Auges unterschiedliche Darstellungen 48a, 48b anzeigen.

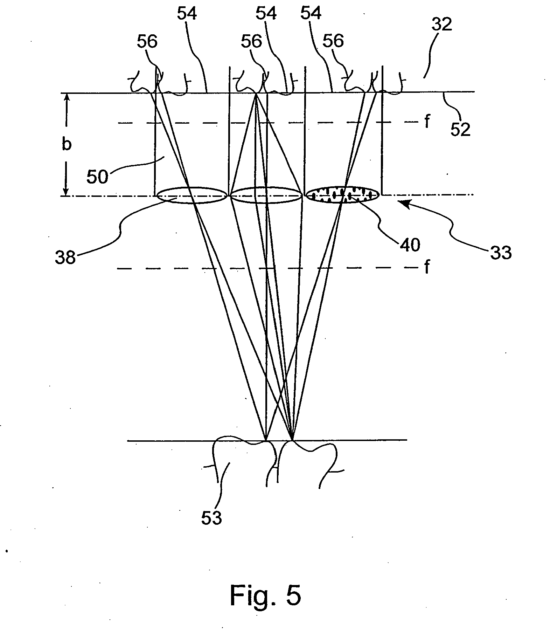

Das Dentalkamerasystem 10 arbeitet wie folgt:
Wie aus Figur 5 ersichtlich, wird der Strahlengang der Optik 33 aufgrund des vorangestellten Linsenarrays 36 in mehrere nebeneinander angeordnete Abbildungskanäle 50 unterteilt, die jeweils eine eigene konventionelle Optik umfassen. Auch eine Erfassungsfläche 52 des Bildwandlers 32 wird entsprechend in mehrere Bildwandlerbereiche 54 unterteilt.

Die Bildweite b zwischen der Erfassungsfläche 52 und dem Linsenarray 36 sowie die Brennweite f der Linsen 36 sind dabei derart gewählt, dass eine.in einem für Dentalkameras üblichen Arbeitsabstand liegende Ebene eines Objekts 53 (hier beispielhaft 12 mm) scharf auf die Erfassungsfläche 50 abgebildet wird. Während einer Aufnahme mit der Dentalkamera 12 werden dann in den Bildwandlerbereichen 54 für die verschiedenen Abbildungskanälen 50 gleichzeitig unterschiedliche Abbildungen 56 des Objekts 53 erfasst, wobei sich die jeweilige Beobachterpositionen und Blickrichtungen entsprechend der Position des Abbildungskanals 50 von Abbildungskanal 50 zu Abbildungskanal 50 geringfügig unterscheiden.

Mit Hilfe des Steuer- und Kommunikationsmoduls 34 werden die einzelnen vom Bildwandler 32 erfassten Abbildungen 56 an die Auswerteeinheit 14 übermittelt. In dieser wird ein plenoptischer Auswertealgorithmus abgearbeitet, welcher anhand der verschiedenen Abbildungen 56 das 4D-Lichtfeld bestimmt.

Aus diesem 4D-Lichtfeld generiert die Auswerteeinheit 14 zwei vollständige Darstellungen 48a, 48b des Objektes 53 mit jeweils unterschiedlichem Beobachterstandpunkt für das linke und rechte Auge und stellt diese auf dem autostereoskopischen Bildschirm 46 dar.

Ferner kann die Auswerteeinheit 14 aus dem 4D-Lichtfeld Darstellungen 48a, 48b mit unterschiedlichen Objektweiten berechnen und aus diesen Darstellungen eine oder bei der 3D-Anzeige zwei scharfe Darstellungen 48a, 48b auswählen. Als Bewertungskriterium für die Auswahl wird hierzu beispielsweise in einem zentralen Teilbereich 60 der Darstellungen 48a, 48b ein Kantenkontrastmessverfahren angewendet, welches über eine Frequenzanalyse den Kontrast und die Dicke von Kanten in den Darstellungen 48a, 48b bewertet. Über ein Optimierungsverfahren, welches die rechnerische Objektweite der Darstellungen 48a, 48b variiert und das Ergebnis des Kantenkontrastmessverfahrens als zu optimierende Größe verwendet, stellt die Auswerteeinheit 14 dann eine nach der eigentlichen Aufnahme stattfindende Autofokusfunktion zur Verfügung.

Die Darstellungen 48a, 48b werden in Echtzeit aktualisiert, sodass auf dem Bildschirm 46 jeweils das gerade von der Dentalkamera 12 erfasste Objekt 53 dargestellt wird. Durch Betätigen des Standbildschalters 26 kann der Benutzer eine Standbildfunktion auslösen, sodass die momentan angezeigten Darstellungen 48a, 48b bis auf weiteres angezeigt und gegebenenfalls archiviert werden.

Über den Moduswahlschalter 28 kann das Dentalkamerasystem 10 in einen 3D-Vermessungsmodus überführt werden. In diesem Betriebsmodus wird ein Tiefenauswertealgorithmus aktiviert, mit dessen Hilfe aus dem 4D-Lichtfeld Tiefeninformationen, wie beispielsweise das Bissprofil eines aufgenommenen Zahns, gewonnen, dargestellt und/oder gespeichert werden. Zur Unterstützung des Tiefenauswertealgorithmus wird die Beleuchtungseinrichtung 24b aktiviert, welche mit Hilfe einer Streifenmaske 62 auf dem Objekt 53 ein Streifenmuster erzeugt.

Ein weiterer über den Moduswahlschalter 28 aktivierbarer Betriebsmodus ist ein Fluoreszenzdetektionsmodus, in welchem zur Erzeugung von Anregungslicht die Beleuchtungseinrichtung 24a, hier in Form einer UV-LED, aktiviert wird. Das so erzeugte Anregungslicht dient dazu, in Kariesbakterien Fluoreszenz anzuregen, sodass Fluoreszenzlicht emittiert wird, welches durch das Eintrittsfenster 22 auf das Linsenarray 36 fällt. Dort werden an den Filterlinsen 40 alle Lichtanteile außer dem Fluoreszenzlicht aus dem Strahlengang weitgehend herausgefiltert, sodass in den zugehörigen Abbildungskanälen 50 nur das Fluoreszenzlicht erfasst wird. Ein Fluoreszenzauswertealgorithmus berücksichtigt dann nur die zu den Filterlinsen 40 gehörigen Abbildungen 56. Auf diese Weise kann ein Kariesbefall lokalisiert dargestellt werden. Mit Hilfe eines Pulsmodus der Beleuchtungseinrichtungen 24a bis 24d und einer entsprechender Auswertung der zu den anderen Linsen 38 gehörigen Abbildungen 56 werden quasi zeitgleich Echtfarb-Darstellungen 48a, 48b des Objekts 53 berechnet und angezeigt, in welchen der über die Fluoreszenzmessung erkannte Kariesbefall beispielsweise durch Falschfarben dargestellt ist.

In Abwandlung zu dem gezeigten Linsenarray 36 mit rechteckiger Rasteranordnung der Linsen 38 können diese auch beispielsweise in einem Hexagonalmuster angeordnet sein.

## Patentansprüche

1. Dentalkamerasystem (10) zum Erzeugen einer Darstellung eines Objektes (53), wobei das Dentalkamerasystem aufweist:
a) eine Dentalkamera (12), die ein Gehäuse (16), eine darin angeordnete Optik (33) und einen Bildwandler (32) aufweist, und
b) eine Auswerteeinheit (14), die mit dem Bildwandler (32) zusammenarbeitet, um das Objekt (53) zu erfassen,
wobei
c) die Optik (33) eine plenoptische Optik ist, die dazu eingerichtet ist, beim Erfassen des Objektes (53) ein 4D-Lichtfeld zu bestimmen,
d) die Auswerteeinheit (14) dazu eingerichtet ist, aus dem 4D-Lichtfeld mindestens eine Darstellung (48a, 48b) des erfassten Objekts (53) zu berechnen,
e) die Optik (33) ein Array (36) von optischen Elementen (38, 40) aufweist, das den Strahlengang der Optik (33) in mehrere nebeneinander angeordnete Abbildungskanäle (52) unterteilt, deren Sichtfelder sich teilweise überlappen,
f) das Array von optischen Elementen (38,40) ein Linsenarray (36) ist, das mehrere Linsen (38, 40) umfasst, wobei jede Linse (38, 40) einem der Abbildungskanäle (52) zugeordnet ist, und
g) die Linsen (36, 40) des Linsenarrays (36) die einzigen Linsen (36, 40) der Optik (33) sind,
**dadurch gekennzeichnet, dass**
h) die Dentalkamera (12) eine Intraoralkamera (12) ist, und
i) einzelne Linsen (36, 40) oder Gruppen von Linsen des Linsenarrays (36) von den anderen Linsen (36, 40) abweichende Brechkraft aufweisen.

2. Dentalkamerasystem nach Anspruch 1, **dadurch gekennzeichnet, dass** die Abbildungskanäle (50) eine erste Gruppe von Abbildungskanälen und eine zweite Gruppe von Abbildungskanälen umfassen und dass im Strahlengang der Abbildungskanäle der ersten Gruppe jeweils ein Filter (40) mit einer ersten Filterwirkung angeordnet ist und im Strahlengang der Abbildungskanäle der zweiten Gruppe kein Filter oder ein Filter mit einer zweiten Filterwirkung angeordnet ist, die sich von der ersten Filterwirkung unterscheidet.

3. Dentalkamerasystem nach Anspruch 2, **dadurch gekennzeichnet, dass** mindestens ein Filter (40) mit der ersten Filterwirkung durch eine Linse (40) des Linsenarrays (36) gebildet wird.

4. Dentalkamerasystem nach einem der vorhergehenden Ansprüche, **dadurch gekennzeichnet, dass** die plenoptische Optik (33) als fokussierte plenoptische Optik (33) ausgebildet ist.

5. Dentalkamerasystem nach einem der vorhergehenden Ansprüche, **dadurch gekennzeichnet, dass** die Auswerteeinheit (14) dazu eingerichtet ist, aus dem 4D-Lichtfeld Darstellungen (48a, 48b) des Objekts (53) mit unterschiedlichen Objektweiten zu berechnen.

6. Dentalkamerasystem nach Anspruch 5, **dadurch gekennzeichnet, dass** die Auswerteeinheit (14) dazu eingerichtet ist, aus den Darstellungen (48a, 48b) mit unterschiedlichen Objektweiten anhand mindestens eines vorgebbaren Bewertungskriteriums automatisch eine Darstellung (48a, 48b) auszuwählen.

7. Dentalkamerasystem nach Anspruch 6, **dadurch gekennzeichnet, dass** das mindestens eine Bewertungskriterium ein Ergebnis einer Kontrastmessung in zumindest einem Teilbereich (60) der Darstellungen (48a, 48b) umfasst.

8. Dentalkamerasystem nach einem der vorhergehenden Ansprüche, **dadurch gekennzeichnet, dass** die Auswerteeinheit (14) dazu eingerichtet ist, aus dem 4D-Lichtfeld Darstellungen (48a, 48b) des Objekts (53) mit unterschiedlichem Beobachterstandpunkt zu berechnen.

9. Dentalkamerasystem nach Anspruch 8, **dadurch gekennzeichnet, dass** die Auswerteeinheit (14) ein 3D-Anzeigegerät, insbesondere einen autostereoskopischen Bildschirm (46), aufweist und dazu eingerichtet ist, unter Verwendung mindestens zwei der Darstellungen (48a, 48b) mit unterschiedlichem Beobachterstandpunkt das erfasste Objekt (53) dreidimensional darzustellen.

10. Dentalkamerasystem nach einem der vorhergehenden Ansprüche, **dadurch gekennzeichnet, dass** die Auswerteeinheit (14) dazu eingerichtet ist, mit Hilfe des 4D-Lichtfeldes das Objekt (53) dreidimensional zu vermessen.

## Claims

1. A dental camera system (10) for generating a representation of an object (53), wherein the dental camera system comprises:
a) a dental camera (12) having a housing (16), an optics (33) arranged therein and an image converter (32), and
b) an evaluation unit (14) that cooperates with the image converter (32) to capture the object (53),
wherein
c) the optics (33) is a plenoptic optics configured to determine a 4D light field upon capturing the object (53),
d) the evaluation unit (14) is configured to calculate at least one representation (48a, 48b) of the captured object (53) from the 4D light field,
e) the optics (33) comprises an array (36) of optical elements (38, 40) which divides the optical path of the optics (33) into several imaging channels (52) arranged next to each other whose fields of view overlap partially,
f) the array of optical elements (38, 40) is a lens array (36) which comprises several lenses (38, 40), wherein each lens (38, 40) being associated with one of the image channels (52), and
g) the lenses (36, 40) of the lens array (36) are the only lenses (36, 40) of the optics (33),
**characterized in that**
h) the dental camera (12) is an intraoral camera (12), and
i) single lenses (36, 40) or groups of lenses of the lens array (36) have diffractive power different from the other lenses (36, 40).

2. The dental camera system according to Claim 2, **characterized in that** the imaging channels (50) comprise a first group of imaging channels and a second group of imaging channels, and **in that** for each imaging channel a filter (40) with a first filter effect is arranged in the light path of the imaging channels of the first group, and no filter, or a filter with a second filter effect which differs from the first filter effect, is arranged in the optical path of the imaging channels of the second group.

3. The dental camera system according to Claim 2, **characterized in that** at least one filter (40) with the first filter effect is formed by a lens (40) of the lens array (36).

4. The dental camera system according to one of the preceding claims, **characterized in that** the plenoptic optics (33) is formed as focused plenoptic optics (33).

5. The dental camera system according to one of the preceding claims, **characterized in that** the evaluation unit (14) is configured to calculate representations (48a, 48b) of the object (53) with different object widths from the 4D light field.

6. The dental camera system according to Claim 5, **characterized in that** the evaluation unit (14) is configured to automatically select a representation (48a, 48b) from the representations (48a, 48b) with different object widths by means of at least one predeterminable evaluation criterion.

7. The dental camera system according to Claim 6, **characterized in that** the at least one evaluation criterion comprises a result of a contrast measurement in at least one subarea (60) of the representations (48a, 48b).

8. The dental camera system according to one of the preceding claims, **characterized in that** the evaluation unit (14) is configured to calculate representations (48a, 48b) of the object (53) with different observer viewpoint from the 4D light field.

9. The dental camera system according to Claim 8, **characterized in that** the evaluation unit (14) comprises a 3D display device, in particular an autostereoscopic screen (46), and is configured to represent the captured object (53) three-dimensionally using at least two of the representations (48a, 48b) with different observer viewpoint.

10. The dental camera system according to one of the preceding claims, **characterized in that** the evaluation unit (14) is configured to measure the object (53) three-dimensionally with the help of the 4D-light field.

## Revendications

1. Système de caméra à usage dentaire (10) destiné à créer une représentation d'un objet (53), le système de caméra à usage dentaire comportant :
a) une caméra dentaire (12), qui comporte un boîtier (16), une optique (33) placée dans ce dernier et un transducteur d'image (32) et
b) une unité d'évaluation (14) qui coopère avec le transducteur d'image (32) pour détecter l'objet (53),
c) l'optique (33) étant une optique plénoptique qui est aménagée pour déterminer lors de la détection de l'objet (53) un champ lumineux en 4D,
d) l'unité d'évaluation (14) étant aménagée pour calculer à partir du champ lumineux en 4D au moins une représentation (48a, 48b) de l'objet (53) détecté,
e) l'optique (33) comportant un réseau (36) d'éléments optiques (38, 40) qui divise la trajectoire du faisceau de l'optique (33) en plusieurs canaux de reproduction (52) placés côte à côte, dont les champs visuels se chevauchent partiellement,
f) le réseau d'éléments optiques (38,40) étant un réseau de lentilles (36) qui comprend plusieurs lentilles (38, 40), chaque lentille (38, 40) étant associée à l'un des canaux de reproduction (52) et
g) les lentilles (36, 40) du réseau de lentilles (36) étant les uniques lentilles (36, 40) de l'optique (33),
**caractérisé en ce que**
h) la caméra dentaire (12) est une caméra intra-orale (12) et
i) des lentilles (36, 40) individuelles ou groupes de lentilles du réseau de lentilles (36) présentent un pouvoir réfringent différent de celui des autres lentilles (36, 40).

2. Système de caméra à usage dentaire selon la revendication 1, **caractérisé en ce que** les canaux de reproduction (50) comprennent un premier groupe de canaux de reproduction et un deuxième groupe de canaux de reproduction et **en ce que** dans la trajectoire du faisceau des canaux de reproduction du premier groupe est placé respectivement un filtre (40) faisant preuve d'un premier effet filtrant et dans la trajectoire du faisceau des canaux de reproduction du deuxième groupe n'est placé aucun filtre ou est placé un filtre faisant preuve d'un deuxième effet filtrant, qui se différencie du premier effet filtrant.

3. Système de caméra à usage dentaire selon la revendication 2, **caractérisé en ce qu'**au moins un filtre (40) faisant preuve du premier effet filtrant est formé par une lentille (40) du réseau de lentilles (36).

4. Système de caméra à usage dentaire selon l'une quelconque des revendications précédentes, **caractérisé en ce que** l'optique (33) plénoptique est réalisée sous la forme d'une optique (33) plénoptique focalisée.

5. Système de caméra à usage dentaire selon l'une quelconque des revendications précédentes, **caractérisé en ce que** l'unité d'évaluation (14) est aménagée pour calculer à partir du champ lumineux en 4D des représentations (48a, 48b) de l'objet (53) à différentes distances de l'objet.

6. Système de caméra à usage dentaire selon la revendication 5, **caractérisé en ce que** l'unité d'évaluation (14) est aménagée pour choisir automatiquement une représentation (48a, 48b) parmi les représentations (48a, 48b) à différentes distances de l'objet, à l'aide d'au moins un critère d'évaluation prédéfinissable.

7. Système de caméra à usage dentaire selon la revendication 6, **caractérisé en ce que** l'au moins un critère d'évaluation comprend un résultat d'une mesure de contraste dans au moins une zone partielle (60) des représentations (48a, 48b).

8. Système de caméra à usage dentaire selon l'une quelconque des revendications précédentes, **caractérisé en ce que** l'unité d'évaluation (14) est aménagée pour calculer à partir du champ lumineux en 4D des représentations (48a, 48b) de l'objet (53) à partir de différentes perspectives de l'observateur.

9. Système de caméra à usage dentaire selon la revendication 8, **caractérisé en ce que** l'unité d'évaluation (14) comporte un appareil d'affichage en 3D, notamment un écran auto-stéréoscopique (46) et est aménagée pour représenter l'objet (53) détecté en trois dimensions, en utilisant au moins deux des représentations (48a, 48b) à partir de différentes perspectives de l'observateur.

10. Système de caméra à usage dentaire selon l'une quelconque des revendications précédentes, caractérisé en ce l'unité d'évaluation (14) est aménagée pour mesurer l'objet 153) en trois dimensions, à l'aide du champ lumineux en 4D.
